# EUROPEAN PATENT APPLICATION

(11) **EP 3 401 917 A1**
(43) Date of publication of application: **14.11.2018**
(21) Application number: 18171122.7
(22) Date of filing: 07.05.2018
(51) Int. Cl.: G16H 40/20

(54) **REMOTE WORKER MONITORING APPARATUS**

(30) Priority: 08.05.2017 GB 201707313
(71) Applicant: Wilson, Stephen, Dumfries & Galloway DG2 0RX (GB); Wilson, Natalia, Dumfries & Galloway DG2 0RX (GB)
(72) Inventor: Wilson, Stephen, Dumfries, Dumfries & Galloway DG2 0RX (GB); Grigorovich, Simeon, Edinburgh, Lothian EH3 9QS (GB); Baker, Lee, Dundee, Tayside DD5 3UU (GB)
(74) Representative: Peter, Kenneth William

(57) **Abstract**

The present invention relates to monitoring apparatus 10 configured to monitor remote workers. The monitoring apparatus 10 comprises main computing apparatus 12 and plural short range wireless transmitters 16 which are each configured to transmit time spaced data packets. Each of the wireless transmitters 16 is installed at a respective different location remote from the main computing apparatus. The monitoring apparatus also comprises plural mobile devices 14 with each of the mobile devices being configured for short range wireless reception of data packets from each of the wireless transmitters 16. Each of the mobile devices 14 is also configured: to record attendance of a worker at each location without worker operation of the mobile device and in dependence on the mobile device being carried by the worker to within wireless communication range of the wireless transmitter 16 at the location whereby the mobile device receives at least one data packet from the wireless transmitter. Each of the mobile devices 14 is further configured to convey each recorded attendance to the main computing apparatus 12.

## Description

### Field of the Invention

The present invention relates to monitoring apparatus configured to monitor remote workers and in particular but not exclusively apparatus configured to monitor care service providers working remotely in the health and social care sectors.

### Background Art

It is known in the health and social care sectors to record attendance of care service providers at homes of care service users. Attendance recordal has benefits for care service provision and management by providing data which may be used for proper scheduling of visits to care service users, allocation of care service resources, onward charging of care service provision and the like. Completion of paper time sheets is burdensome. Analysis of completed time sheets and distillation of useful information from the completed time sheets are also burdensome, in particular when many time sheets are involved. According to a known approach involving application of technology, the care service provider uses either an App on his or her smartphone or the care service user's telephone to log in at the start of a care visit, record pertinent information and log out at the end of the care visit. A disadvantage of this known approach is the need for the care service provider to remember or be minded to operate the App or the care service user's telephone.

The present invention has been devised in light of the inventors' appreciation of the above mentioned shortcoming of the known approach to recording attendance of care service providers. It is therefore an object for the present invention to provide improved monitoring apparatus which is configured to monitor remote workers. It is a further object for the present invention to provide an improved method for monitoring remote workers.

### Statement of Invention

According to a first aspect of the present invention there is provided monitoring apparatus configured to monitor remote workers, the monitoring apparatus comprising:
main computing apparatus;
plural short range wireless transmitters which are each configured to transmit time spaced data packets, each of the wireless transmitters being installed at a respective different location remote from the main computing apparatus; and
plural mobile devices, each of the mobile devices being configured for short range wireless reception of data packets from each of the wireless transmitters,
each of the mobile devices being further configured: to record attendance of a worker at each location without worker operation of the mobile device and in dependence on the mobile device being carried by the worker to within wireless communication range of the wireless transmitter at the location whereby the mobile device receives at least one data packet from the wireless transmitter; and to convey each recorded attendance to the main computing apparatus.

The monitoring apparatus is configured to monitor remote workers such as care service providers working remotely in the health and social care sectors. The monitoring apparatus comprises main computing apparatus, such as a central server operated by or on behalf of a care service provider organisation. The monitoring apparatus further comprises plural short range wireless transmitters with each wireless transmitter being installed at a respective different location remote from the main computing apparatus, such as in the home of each of many care service users. Each short range wireless transmitter is configured to transmit time spaced data packets. The monitoring apparatus yet further comprises plural mobile devices with each mobile device being configured for short range wireless reception of data packets from each of the wireless transmitters. The mobile device may be a smartphone or a tablet computer. The mobile device may comprise a short range wireless receiver and more usually a short range wireless transceiver.

Each of the mobile devices is further configured, such as by way of an App running on the mobile device, to record attendance of a worker at each location without worker operation of the mobile device and in dependence on the mobile device being carried by the worker to within wireless communication range of the wireless transmitter at the location whereby the mobile device receives at least one data packet from the wireless transmitter. The known approach depends on the worker remembering or being minded to operate the App or the care service user's telephone. In contrast, the present approach is automatic in that attendance is recorded in dependence on the mobile device being carried by the worker to within wireless communication range of the wireless transmitter and without worker operation of the mobile device. The mobile device is further configured to convey each recorded attendance to the main computing apparatus. The mobile device may be configured to convey the content of a received data packet to the main computing apparatus. The content of a data packet is described further below.

Each of the mobile devices is configured to record attendance of the worker at each location in dependence on the mobile device being carried by the worker to within wireless communication range of the wireless transmitter at the location. Attendance of the worker is recorded further in dependence on the mobile device receiving at least one data packet from the wireless transmitter. Attendance may be recorded in dependence on the mobile device receiving at least one data packet from the wireless transmitter and not in dependence on a positioning system, such as a GPS receiver comprised in the mobile device or triangulation of position of the mobile device by way of cellular telephone towers. However, and as described further below, geolocation data may be generated in dependence on the mobile device receiving at least one data packet from the wireless transmitter by, for example, associating a unique identifier for the wireless transmitter with a geographic location for the wireless transmitter. Each data packet may therefore comprise a unique identifier for the wireless transmitter. For example, where the wireless transmitter is a Bluetooth beacon the unique identifier may comprise a universally unique identifier (UUID).

The identity of each of the plural wireless transmitters may be stored in the main computing apparatus. More specifically, a unique identifier for each of the plural wireless transmitters may be stored in the main computing apparatus. The unique identifier may, for example, be a UUID, a media access control (MAC) address or a service set identifier (SSID). The step of conveying each recorded attendance to the main computing apparatus may comprise conveying the unique identifier for the wireless transmitter to the main computing apparatus. The main computing apparatus may comprise a database, such as a look up table, which provides for correspondence between each stored unique identifier and data pertaining to the location of the wireless transmitter. The data pertaining to the location of the wireless transmitter may comprise data for a person at the location, such as a care service receiver who is resident at the location. The data for the person at the location may comprise at least one of location, such as an address, and a name of the person.

Each short range wireless transmitter and each mobile device may be configured for communication of data packets from the transmitter to the mobile device. More specifically, each short range wireless transmitter and each mobile device may be configured for communication of data by packet switching. In a form of the invention, communication between a short range wireless transmitter and a mobile device may be in accordance with the Bluetooth standard and more specifically the Bluetooth low energy (or Bluetooth Smart) standard. The short range wireless transmitter may be a Bluetooth beacon. The short range wireless transmitter may therefore be an always on transmitter. Furthermore, the mobile device may be configured for communication in accordance with the Bluetooth standard. Each short range wireless transmitter and each mobile device may therefore form an ad hoc wireless personal area network (WPAN). Each of the wireless transmitter and the mobile device may be configured for wireless communication over a range consistent with a WPAN. Attendance recordal may thus be achieved when the mobile device is carried by the worker within 10 m of the short range wireless transmitter and typically when the mobile device is carried by the worker into the same room as the short range wireless transmitter.

Each mobile device may be configured to record when the mobile device is brought into wireless range of the short range wireless transmitter. Recordal of when the mobile device is brought into wireless range of the short range wireless transmitter may reflect arrival of the worker at the location. Furthermore, arrival of the worker at the location may be reflected by reception by the mobile device of a first data packet from the short range wireless transmitter. The mobile device may be configured to determine at least one of a time and a date of when the mobile device is brought into wireless range of the short range wireless transmitter. When the mobile device is brought into wireless range of the short range wireless transmitter, attendance recordal data may be conveyed to the main computing apparatus. Following recordal of when the mobile device is brought into wireless range of the short range wireless transmitter, each mobile device may be configured to record when the mobile device moves out of wireless range of the short range wireless transmitter. The mobile device may be configured to determine when it ceases to receive data packets from the wireless transmitter and to thereby determine that the mobile device has moved out of wireless range of the short range wireless transmitter. Recordal of when the mobile device moves out of wireless range of the short range wireless transmitter may reflect departure of the worker from the location. When the mobile device moves out of wireless range of the short range wireless transmitter, departure recordal data may be conveyed to the main computing apparatus.

Alternatively or in addition, when the mobile device is within wireless range, the mobile device may be operative to determine distance between the mobile device and the wireless transmitter. Distance may be determined in dependence on strength of wireless signal received by the mobile device from the wireless transmitter, for example received signal strength indication (RSSI). Each data packet transmitted by the wireless transmitter may comprise a signal strength value, such as an RSSI value, for the wireless transmitter. The mobile device may be configured to determine the distance between the wireless transmitter and the mobile device in dependence on the signal strength value comprised in a data packet and the strength of the wireless signal received by the mobile device from the wireless transmitter. The mobile device may be operative to record attendance at the location, such as by way of sending of attendance recordal data, when the determined distance is less than a threshold value, such as less than 3 m. Alternatively or in addition, determined distance may be conveyed to the main computing apparatus.

The mobile device may be configured to convey recorded attendance to the main computing apparatus in real or near real time. A database comprised in the main computing apparatus may thus be kept up to date with the progress of the worker carrying the mobile device. The mobile device may be configured to convey the recorded attendance to the main computing apparatus by way of a wide area wireless communication channel, such as by way of a cellular communication channel.

The mobile device may be configured to convey each recorded attendance to the main computing apparatus along with an identifier which is unique to at least one of the mobile device, such as the IMEI number, and the worker, such as a staff number. A database comprised in the main computing apparatus may therefore contain data which records attendance for each of plural workers.

The main computing apparatus may comprise a database containing an attendance schedule for at least one worker. The main computing apparatus may be configured to compare recorded attendances received from a mobile device with the attendance schedule and to make a determination in respect of compliance with the attendance schedule in dependence on the comparison. The main computing apparatus may be operative to generate an alarm signal if there is determined to be lack of compliance with the attendance schedule. Where the worker is a care service provider, monitoring for lack of compliance with an attendance schedule may be important when attendance and perhaps timely attendance is required for the wellbeing of a care service user.

The wireless transmitter may comprise relative position detecting apparatus, such as an accelerometer and more specifically a 3D accelerometer. The relative position detecting apparatus may be operative to determine a relative position of the wireless transmitter such as a relative position in 3D space. The wireless transmitter may be put in a specific location. More specifically, the wireless transmitter may be operated to record the specific location as a reference location, such as by operating the wireless transmitter to set an output from the accelerometer to zero. Movement of the wireless transmitter from the specific location may be detected by the relative position detecting apparatus with an output from the relative position detecting apparatus corresponding to extent of movement of the wireless transmitter from the specific location. The wireless transmitter may be configured to determine the moved location of the wireless transmitter relative to the reference location in dependence on the output from the relative position detecting apparatus. A data packet transmitted by the wireless transmitter may comprise location data, such as moved location data. The mobile device may be operative to record attendance at the location when a value comprised in the moved location data is more than a predetermined distance value. The predetermined distance may reflect opening of a door when the wireless transmitter is attached to the door. Furthermore, the mobile device may be configured to convey the attendance recordal data to the main computing apparatus when the value comprised in the moved location data is more than the predetermined distance value. Alternatively or in addition, the mobile device may be configured to convey the location data to the main computing apparatus. By way of example, when the wireless transceiver is mounted on a movable object at the location, such as a door, movement of the movable object may be detected and the attendance recordal data may be conveyed to the main computing apparatus in dependence on the detected movement. Activity of the worker may thus be monitored. For example, a care service user leaving from or returning to a room within a property may be detected whereby compliance with an attendance schedule comprised in the main computing apparatus may be monitored.

The main computing apparatus may comprise central computing apparatus operated by the care service provider organisation. The like of review and analysis of recorded attendances may be performed by way of the central computing apparatus. The main computing apparatus may comprise a cloud computing arrangement. Data, such as of recorded attendances, may be stored in the cloud computing arrangement.

The monitoring apparatus may be care service monitoring apparatus. More specifically the monitoring apparatus may be home care monitoring apparatus.

According to a second aspect of the present invention there is provided a method for monitoring remote workers, the method comprising:
providing each of plural short range wireless transmitters at a respective different location remote from main computing apparatus, each short range wireless transmitter being configured to transmit time spaced data packets; and
carrying of each of plural mobile devices by a worker to within wireless communication range of one of the plural wireless transmitters, each of the mobile devices being configured for short range wireless reception of data packets from each of the wireless transceivers, each of the mobile devices being further configured: to record attendance of a worker at the location without worker operation of the mobile device and in dependence on the mobile device being carried by the worker to within wireless communication range of the wireless transmitter at the location whereby the mobile device receives at least one data packet from the wireless transmitter; and to convey each recorded attendance to the main computing apparatus.

Embodiments of the second aspect of the present invention may comprise one or more features of the first aspect of the present invention.

According to a further aspect of the present invention there is provided monitoring apparatus configured to monitor remote workers, the monitoring apparatus comprising: main computing apparatus; a short range wireless transmitter installed at a location remote from the main computing apparatus; and a mobile device configured for wireless communication with the wireless transmitter, the mobile device being further configured: to record attendance of a worker at the location in dependence on the mobile device being carried by the worker to within wireless communication range of the wireless transmitter at the location; and to convey a recorded attendance to the main computing apparatus. Embodiments of the further aspect of the present invention may comprise one or more features of the first aspect of the present invention.

Remote monitoring of a person to detect adverse health events is known. The present inventors became appreciative of the benefit of anticipating the onset of adverse health events. Therefore and in another aspect the present invention relates to use of at least one biometric sensor to predict an adverse outcome concerning health of a person.

According to a third aspect of the present invention there is provided monitoring apparatus comprising:
main computing apparatus; and
a biomedical sensor arrangement worn by a person at a location remote from the main computing apparatus, the monitoring apparatus being configured to convey a signal generated by the biomedical sensor arrangement to the main computing apparatus,
the main computing apparatus comprising a predictive model, the main computing apparatus being operative in dependence on the signal received from the biomedical sensor arrangement and the predictive model to predict an adverse outcome concerning health of the person.

The monitoring apparatus comprises main computing apparatus and a biomedical sensor arrangement worn by a person at a location remote from the main computing apparatus, such as in the person's home. The biomedical sensor arrangement may be configured to be worn by the person. More specifically, the biomedical sensor arrangement may be configured to measure at least one bodily function of the person when the biomedical sensor arrangement is worn. The monitoring apparatus is configured to convey a signal generated by the biomedical sensor arrangement to the main computing apparatus. For example, the generated signal is conveyed by way of a cellular communication channel. The main computing apparatus comprises a predictive model. The main computing apparatus is operative in dependence on the signal received from the biomedical sensor arrangement and the predictive model to predict an adverse outcome concerning health of the person. More specifically the main computing apparatus may be operative to predict the onset of an adverse health event of the person, such as a seizure.

Known approaches involve monitoring a person and detecting an adverse health event of the person after it has occurred. Action to address the adverse health event after onset is usually less effective and involves more resources than acting before onset of the adverse health event. The present approach involves use of a biomedical sensor arrangement which is worn by a person and is operative to measure at least one bodily function of the person, such as at least one vital sign of the person. The biomedical sensor arrangement may be operative to generate a signal which corresponds to the at least one measured bodily function. Furthermore, the present approach involves main computing apparatus being operative in dependence on the generated signal and a predictive model to predict an adverse outcome concerning health of the person. The predictive model may be configured to relate the at least one measured bodily function conveyed by the generated signal to risk of the person developing one or more adverse health events. More specifically and as described further below, the predictive model may be configured in dependence on data which relates measurement of bodily function to health of the person. For example, the biomedical sensor arrangement may be operative to measure body temperature, pulse rate and galvanic skin response and the main computing apparatus may be operative in dependence on the predictive model to determine that a particular combination of body temperature, pulse rate and galvanic skin response measurements reflects a deviation from normal health for the person and is therefore indicative of a future adverse health event, such as a seizure.

The biomedical sensor arrangement may comprise at least one biomedical sensor. The biomedical sensor may be a physical sensor such as a heart rate monitor, an ECG monitor, a thermometer, a galvanic skin response sensor, a blood pressure monitor or a blood oxygen monitor. The biomedical sensor arrangement may comprise a short range wireless transmitter, the biometric sensor arrangement being configured to wirelessly transmit from the short range wireless transmitter data corresponding to measurements made by way of at least one biomedical sensor. The short range wireless transmitter may be configured for operation in accordance with the Bluetooth standard and more specifically the Bluetooth low energy (or Bluetooth Smart) standard.

The monitoring apparatus may further comprise a mobile device which is configured for short range wireless reception. The mobile device may therefore comprise a short range wireless receiver and more specifically a short range wireless transceiver. The mobile device may be configured for short range wireless reception from the biomedical sensor arrangement. A short range wireless receiver of the mobile device may therefore be configured for operation in accordance with the Bluetooth standard. The mobile device may be a smartphone or a tablet computer. Where the mobile device is a smartphone, the smartphone may be carried in the like of a pocket of the person being monitored whereby the mobility of the person being monitor is not limited.

The monitoring apparatus may comprise a movement monitor which is configured to be worn by the person. The movement monitor may comprise at least one of an accelerometer, such as a 3D accelerometer, and a gyroscope. The movement monitor may be configured to be supported on the person such as in a pocket of clothing worn by the person. The movement monitor may be comprised in the mobile device.

The monitoring apparatus may be configured to convey signals generated by the biomedical sensor to the main computing apparatus by way of a wireless wide area network communication channel. The wireless wide area network communication channel may comprise a cellular communication channel. The mobile device may comprise a cellular communication transceiver. Where the mobile device is a smartphone, a cellular communication channel may be established by way of a cellular communication transceiver comprised in the smartphone. The cellular communication transceiver may, for example, be operative in accordance with the GPRS or 4G standard.

As described above, the predictive model may be configured in dependence on data which relates measurements of bodily function to health of the person. The predictive model may comprise a database containing data which relates measurements of bodily function to at least one health state of the person, such as well, mostly well, moderately well, mostly unwell and unwell. The database may be formed and configured before use of the monitoring apparatus, such as in dependence on measurement data from a plurality of subjects which corresponds to what is deemed to reflect normal health for the plurality of subjects. Alternatively or in addition, the database may be formed and configured during use of the monitoring apparatus when it is known that the person wearing the biomedical sensor arrangement is in a particular health state, such as normal health.

The predictive model may be configured for one of different classes of persons. A class of persons may be defined by at least one common attribute, such as male or female, age group or ethnicity. A range of values for measurements of bodily function which are deemed to be normal may vary from one class of persons to another. For example, an upper limit for normal blood pressure may be lower for a first class of younger age group than for a second class of older age group. The predictive model may be configured according to the class of persons to which the wearer of the biomedical sensor arrangement belongs. Nevertheless, the predictive model may be configured to take account of measurements which are deemed to reflect at least one health state for the wearer.

The predictive model may be configured in dependence on a machine learning approach and more specifically a machine learning algorithm. The machine learning approach may be supervised. A machine learning approach may adapt the predictive model to at least one of a class of persons and a particular user. Furthermore, a machine learning approach may adapt the predictive model as the user's bodily function characteristics change over time. The likelihood of false prediction may be thus reduced and accuracy of prediction may be increased. Different machine learning approaches were considered by the inventors.

A bodily function may vary over what is deemed to be a normal range with extent of variation outside the normal range being indicative to a lesser or greater extent of risk of onset of an adverse health event. There may therefore be a distribution of values for a bodily function, with the probative worth of where a measurement falls within the distribution depending on sufficient and appropriate data being comprised in the predictive model. Furthermore, more effective prediction may be achieved when prediction is in dependence on measurement of a plurality of different bodily functions. The inventors initially used linear and logistic regression models for modelling health of the user. However, the linear and logistic regression models were found to have shortcomings. Firstly, discrimination in linear and logistic regression models is linear whereas some of the bodily functions exhibit non-linear behaviour. Secondly, regression modelling is susceptible to noise with noise being an issue for the present approach in certain circumstances. The inventors therefore used the k-nearest neighbours (k-NN) algorithm to model the health of the user. The k-NN algorithm was found to provide for improved modelling over regression modelling. Thereafter the inventors used an artificial neural network (ANN) and more specifically a supervised ANN. The ANN was found to provide for improved modelling over k-NN modelling. The predictive model may therefore comprise an ANN and more specifically a supervised ANN. The ANN may be configured before use in dependence on training data formed from measurements made in respect of a group of persons and more specifically persons belong to a particular class of persons. Alternatively or in addition, the ANN may be operative in dependence on the signal generated by the biomedical sensor arrangement to configure or modify the predictive model. Configuration or modification of the predictive model may depend on the wearer of the biomedical sensor arrangement being in a known state of health, such as normal health, when measurements made by the biomedical sensor arrangement are used to configure or modify the predictive model.

The main computing apparatus may be configured to generate an alarm in dependence on prediction of an adverse outcome concerning health of the person.

The main computing apparatus may comprise output apparatus which is operable to output the alarm in at least one human perceptible form. The output apparatus may be configured to output the alarm in at least one of: visible form, audible form and readable form. The output apparatus may therefore comprise at least one of electroacoustic apparatus, such as a loudspeaker, a light source, such as a light emitting device (LED) or a visual display unit, and hard copy generating apparatus, such as a printer.

According to a fourth aspect of the present invention there is provided a monitoring method comprising:
wearing of a biomedical sensor arrangement by a person at a location remote from main computing apparatus; and
operating of the main computing apparatus to predict an adverse outcome concerning health of the person in dependence on a signal generated by the biomedical sensor arrangement and a predictive model.

Embodiments of the fourth aspect of the present invention may comprise one or more features of the third aspect of the present invention.

According to a yet further aspect of the present invention there is provided monitoring apparatus comprising: main computing apparatus; and a biomedical sensor arrangement worn by a person at a location remote from the main computing apparatus, the monitoring apparatus being configured to convey signals generated by the biomedical sensor arrangement to the main computing apparatus, the main computing apparatus being configured to make a determination in respect of health of the person in dependence on signals received from the biomedical sensor arrangement. Embodiments of the yet further aspect of the present invention may comprise one or more features of the third aspect of the present invention.

### Brief Description of Drawings

Further features and advantages of the present invention will become apparent from the following specific description, which is given by way of example only and with reference to the accompanying drawings, in which:

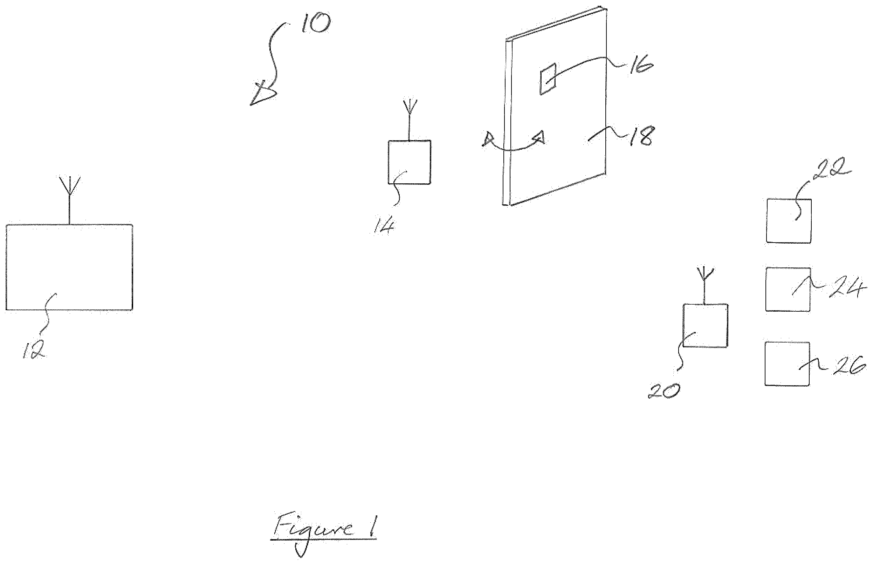

Figure 1 is a representation of monitoring apparatus according to embodiments of the present invention.

### Description of Embodiments

A representation of monitoring apparatus 10 according to embodiments of the present invention is shown in Figure 1. A first embodiment of monitoring apparatus comprises main computing apparatus 12, which is of conventional form and function except as described hereinbelow, a smartphone 14 (which constitutes a mobile device), which is of conventional form and function except as described hereinbelow, and a Bluetooth beacon 16 (which constitutes a wireless transmitter), which is operative in accordance with the Bluetooth low energy standard, such as a Beacon from Kontakt.io of Marienstraße 10, 10117 Berlin, Germany. The Bluetooth beacon 16 comprises a 3D accelerometer. The main computing apparatus 12 is operated by or on behalf of a care service provider organisation and is configured for wide area wireless communication by way of a cellular communication channel in accordance with the GPRS standard, 4G standard or the like. The main computing apparatus 12 also comprises cloud based data storage. The smartphone 14 is carried by a care service provider and is configured for wireless communication by way of each of a cellular communication channel in accordance with the GPRS standard, 4G standard or the like and a Bluetooth channel in accordance with the Bluetooth low energy standard. In practice, the care service provider organisation has many care service providers reporting to it whereby there are many care service providers each carrying a smartphone 14. The Bluetooth beacon 16 is attached to the inside of a door 18 in the home of a care service user. In practice, the care service provider organisation has responsibility for provision of care services to many homes whereby each of the many homes has a Bluetooth beacon 16 installed.

A second embodiment of monitoring apparatus comprises the main computing apparatus 12, a further smartphone 20, (which constitutes a mobile device), which is of conventional form and function except as described hereinbelow, a heart rate monitor 22, such as a Cosinuss One from Cosinuss GmbH of Kistlerhofstr. 60, D-81379 München, Germany, an ECG monitor 24, such as a Qardiocore from Qardio Europe Ltd., 14-16 Dowgate Hill, London, EC4R 2SU, United Kingdom, and a thermometer 26. The thermometer 26 forms part of the Cosinuss One heart rate monitor 22. The further smartphone 20 comprises a 3D accelerometer and a gyroscope in accordance with usual smartphone design. The 3D accelerometer and the gyroscope together constitute a movement monitor. The further smartphone 20, the heart rate monitor 22, the ECG monitor 24 and the thermometer 26 together constitute a biomedical sensor arrangement. Each of the further smartphone 20, the heart rate monitor 22, the ECG monitor 24 and the thermometer 26 is configured for wireless communication in accordance with the Bluetooth low energy standard. The further smartphone 20 is further configured for cellular communication in accordance with the GPRS standard, 4G standard or the like. Each of the heart rate monitor 22, the ECG monitor 24 and the thermometer 26 is configured to be worn by a person being monitored in their home, such as on the wrist, on the arm, around the chest or on the head.

Operation of each of the first and second embodiments will now be described with reference to Figure 1.

The first embodiment is operative when in use to monitor a remotely working care service provider. Prior to use of the first embodiment, a Bluetooth beacon 16 is installed in the home of each care service user by attaching the Bluetooth beacon to a door in the home of the care service user. Typically, the Bluetooth beacon 16 is attached to the inside of the door of the room in which the care service user is accustomed to use. The door to which the Bluetooth beacon 16 is attached is closed and the Bluetooth beacon is operated to record the present location of the Bluetooth beacon as a reference location. The person installing the Bluetooth beacon 16 records user information including the name of the user, the user's address including floor level if appropriate, the location of the beacon within the property, e.g. inside front door, and the GPS coordinates of the beacon by way of a smartphone carried by the installer which comprises a GPS receiver. Furthermore, a database of the main computing apparatus is configured with an attendance schedule for each of all the care service providers reporting to the care service provider business. The user information collected for each user by the installer is conveyed to the main computing apparatus and included in a record for the user in the database. Each attendance schedule is indexed by way of a unique identifier for the care service provider, such as the care service provider's employee number or a unique identifier for the smartphone 14 carried by the care service provider, such as the IMEI number for the smartphone. An attendance schedule comprises a date and time ordered list of records with each record relating to a care service user to be attended by the care service provider. Each record contains the name, address and other information of the care service user, the universally unique identifier (UUID) for the Bluetooth beacon 16 installed in the care service user, the scheduled date and time for arrival with the care service user, the scheduled duration of attendance at the care service user and any other pertinent information, such as tasks to be performed while attending the care service user. Records within an attendance schedule are indexed by way of the UUID.

During use of the first embodiment, the care service provider arrives at the home of the care service user. The Bluetooth transmitter comprised in the Bluetooth beacon 16 has a range of 10 m and is operative to transmit time spaced data packets. Each data packet comprises the UUID for the Bluetooth beacon 16 and a received signal strength indication (RSSI) value for signals transmitted by the Bluetooth transmitter. When the care service provider is within wireless range of the Bluetooth transmitter, the smartphone 14 carried by the care service provider is operative to receive at least one data packet with an App running on the smartphone putting the smartphone into a listening mode. The App is operative in dependence on reception of the data packet to register attendance of the care service provider. In addition, the App is operative to convey attendance recordal data to the main computing apparatus 12 by way of the cellular communication channel between the smartphone 14 and the main computing apparatus 12. The App is operative of its own accord to operate in this fashion and without care service provider operation is respect of the smartphone 14 other than to have the smartphone turned on. The attendance recordal data comprises the unique identifier for the care service provider, which is an intrinsic part of the smartphone 14 or is stored in the smartphone, the UUID comprised in the received data packet and the date and time of reception of the data packet. The main computing apparatus 12 is operative to access the attendance schedule for the care service provider in dependence on the unique identifier for the care service provider comprised in the attendance recordal data. The main computing apparatus 12 is further operative to access the record relating to a care service user in dependence on the UUID comprised in the attendance recordal data. The record is amended to reflect attendance of the care service provider at the care service user. The main computing apparatus is configured to analyse attendance schedules for compliance. Frequency and timing of analysis of attendance schedules is determined in accordance with care service provision requirements. For example, analysis may be performed for compliance with attendance to within one hour in respect of care service visits involving administering medicine of a critical nature to care service users. By way of another example, analysis may be performed for compliance with attendance within a four hour shift for care service providers when care service visits involve attending to less critical needs of care service users. Where analysis reveals there to be lack of compliance within a predetermined time limit, the main computing apparatus is operative to generate an alarm for the care service provider organisation.

In a first form of this embodiment, the App running on the smartphone 14 is operative to determine a distance between the smartphone and the Bluetooth beacon 16 in dependence on the RSSI value comprised in the data packet received from the Bluetooth beacon and the RSSI of the data packet as received from the Bluetooth beacon. Extent of loss of signal strength between transmission and reception provides for determination of distance. The App then compares the determined distance with a predetermined distance, such as 3 m, and then only conveys the attendance recordal data to the main computing apparatus 12 when the determined distance is less than the predetermined distance. The App is thus operative to record attendance when the care service provider is within the predetermined distance of the Bluetooth beacon. Recording attendance based on a smaller distance than the range of the Bluetooth beacon 16 may be appropriate in certain circumstances. For example, the care service user may be living in a residential home occupied by several care service users each of whom is provided with a Bluetooth beacon 16 and all of whom live within Bluetooth beacon range of each other.

Upon arrival at the home of the care service user, the care service provider opens the door 18 of the room in which the care service user is present whereby the Bluetooth beacon 16 on the inside of the door moves. The 3D accelerometer comprised in the Bluetooth beacon 16 is operative to record movement of the Bluetooth beacon as the door is opened. In a second form of this embodiment, the App running on the smartphone 14 is operative to compare the output signal from the 3D accelerometer with a threshold movement value which corresponds to the door being opened and to convey the attendance recordal data to the main computing apparatus 12 only when the output signal exceeds the threshold movement value. Recording attendance based on movement of the Bluetooth beacon 16 may be appropriate in certain circumstances. For example, the care service user may be living in a residential home occupied by several care service users each of whom is provided with a Bluetooth beacon 16 and all of whom live within Bluetooth beacon range of each other whereby the second form of the embodiment constitutes an alternative or additional approach to the first form of the embodiment.

During the care service visit, the care service provider carries out the tasks required of the visit. The App is configured to display the tasks required of the visit with completion of each task being recorded by way of the App by the care service provider operating the smartphone 14, such as by ticking a box displayed on the screen of the App. Completion of tasks is conveyed from the smartphone 14 to the main computing apparatus 12 by way of the cellular communication channel. At the end of the care visit, the care service provider moves out of range of the Bluetooth beacon 16 and therefore the smartphone 14 ceases to receive data packets from the Bluetooth beacon. The App is operative to monitor for continued receipt of data packets from the Bluetooth beacon and upon ceasing to receive data packets is operative to register ceasing of receipt of data packets. The App is then further operative to provide for departure recordal data to be conveyed from the smartphone 14 to the main computing apparatus 12 by way of the cellular communication channel. The departure recordal data comprises the unique identifier for the care service provider, the UUID comprised in the last received data packet and the date and time of ceasing of reception of the data packets. The main computing apparatus 12 is then operative to access the appropriate record comprised in the database as described above and to compare the departure recordal data with data comprised in the record for compliance. For example, compliance may involve the care service visit to be of at least 15 minutes duration. The main computing apparatus 12 is then operative to update the record accordingly. The main computing apparatus 12 is operative to analyse attendance schedules for compliance, with frequency and timing of analysis being determined in accordance with care service provision requirements. In a form of this embodiment, the App is operative to convey the departure recordal data to the main computing apparatus 12 when the distance between the smartphone 14 and the Bluetooth beacon 16 is more than a predetermined distance consistent with the process described above with reference to the first form of the embodiment. In another form of this embodiment, the App is operative to convey the departure recordal data to the main computing apparatus 12 when opening and closing of the door 18 upon departure has been detected by way of the 3D accelerometer comprised in the Bluetooth beacon 16 consistent with the process described above with reference to the second form of the embodiment.

The second embodiment is operative when in use to monitor plural bodily functions of a person and to predict the onset of an adverse health event of the person in dependence the monitored bodily functions and a predictive model. The predictive model is comprised in the main computing apparatus 12. The predictive model is a supervised artificial neural network (ANN) model. Prior to use of the second embodiment, the ANN is therefore trained with training data consisting of a set of training examples, with each training example being a pair consisting of an input object and a desired output object. Input objects are formed in dependence on combination of heart rate, ECG, temperature and movement measurements. Output objects consist of a set of different health status indications, i.e. well, mostly well, moderately well, mostly unwell and unwell. Training takes place in two stages.

A first stage involves training based on a class of persons to which the intended user belongs. For example, the appropriate class may be males in the seventy to eighty age group. Heart rate, ECG, temperature and movement are measured for a significant number of persons in the appropriate class when the persons are of different known health status. The measurements are normalised such that measurements of each bodily function have zero mean and unit variance. Then combinations of the normalised measurements are associated with each known health status to thereby form training examples which are applied to the ANN model to thereby configure the ANN model for the appropriate class of persons.

The second stage of training involves training based on the user himself to adapt the class based model to the user. Heart rate, ECG, temperature and movement are measured for the user when the user is of different known health status. The measurements are made with the heart rate monitor 22, the ECG monitor 24, the thermometer 26 and the movement monitor comprised in the further smartphone 20. Again, the measurements are normalized before combinations of the normalised measurements are associated with each known health status to thereby form training examples which are applied to the ANN model to thereby adapt the ANN model for the user. The second stage of training is performed during initial use of the second embodiment and periodically thereafter at appropriate intervals to take account of changes over time in respect of behaviour and physiology of the user which affect the monitored bodily functions vis-à-vis the different known health status.

The second embodiment is brought into use by the user wearing the heart rate monitor 22, the ECG monitor 24 and the thermometer 26 and supporting the further smartphone 20 at a location on the upper half of the torso such as in a shirt pocket. Locating the further smartphone 20 in this way provides for monitoring of movement which is liable to reveal aberrant gait patterns which are indicative of a health problem. Measurements made by the heart rate monitor 22, the ECG monitor 24 and the thermometer 26 are transmitted to the further smartphone 20 by way of the Bluetooth communication channel between each measurement device and the further smartphone. The further smartphone 20 is then operative to convey the measurements received from the measurement devices and from the movement monitor to the main computing apparatus 12 by way of the cellular communication channel along with a unique identifier for the user, such as the IMEI number for the further smartphone. The unique identifier for the user is used by the main computing apparatus 12 to access the ANN model for the user from amongst other ANN models for other users. The main computing apparatus is then operative to apply combinations of the measured data to the ANN model as input objects and to analyse the output objects generated in response to the input objects. Analysis involves detecting moderately well and mostly unwell output objects and generating an investigation needed alarm for supervisory personnel. Moderately well and mostly unwell output objects reflect a decline in the health of the user which are indicative of risk of onset of an adverse health event and thus merit investigation. Analysis also involves detecting unwell output objects and generating a critical alarm for supervisory personnel. An unwell output object reflects a severe decline in the health of the user which necessitates prompt action. In certain circumstances, the health of a person may decline to unwell suddenly and without progressing through indicative stages. The present approach is therefore configured to respond accordingly.

## Claims

1. Monitoring apparatus configured to monitor remote workers, the monitoring apparatus comprising:
main computing apparatus;
plural short range wireless transmitters which are each configured to transmit time spaced data packets, each of the wireless transmitters being installed at a respective different location remote from the main computing apparatus; and
plural mobile devices, each of the mobile devices being configured for short range wireless reception of data packets from each of the wireless transmitters,
each of the mobile devices being further configured: to record attendance of a worker at each location without worker operation of the mobile device and in dependence on the mobile device being carried by the worker to within wireless communication range of the wireless transmitter at the location whereby the mobile device receives at least one data packet from the wireless transmitter; and to convey each recorded attendance to the main computing apparatus.

2. Monitoring apparatus according to claim 1 wherein a data packet received from the wireless transmitter comprises a unique identifier for the wireless transmitter, the step of conveying each recorded attendance to the main computing apparatus comprises conveying the unique identifier for the wireless transmitter to the main computing apparatus, and a unique identifier for each of the plural wireless transmitters is stored in the main computing apparatus.

3. Monitoring apparatus according to claim 2 wherein the main computing apparatus comprises a database, which provides for correspondence between each stored unique identifier and data pertaining to the location of the respective wireless transmitter.

4. Monitoring apparatus according to any one of the preceding claims wherein each wireless transmitter and each mobile device are configured for communication of data packets from the transmitter to the mobile device by packet switching.

5. Monitoring apparatus according to claim 4 wherein the wireless transmitter is an always on transmitter.

6. Monitoring apparatus according to any one of the preceding claims wherein each of the wireless transmitter and the mobile device is configured for wireless communication over a range consistent with a wireless personal area network (WPAN) whereby attendance recordal is achieved when the mobile device is carried by the worker within 10 m of the wireless transmitter.

7. Monitoring apparatus according to any one of the preceding claims wherein the mobile device is configured to determine in dependence on reception by the mobile device of the first data packet from the wireless transmitter at least one of a time and a date when the mobile device is brought into wireless range of the wireless transmitter.

8. Monitoring apparatus according to claim 7 and following recordal of when the mobile device is brought into wireless range of the wireless transmitter, the mobile device is configured: to determine when it ceases to receive data packets from the wireless transmitter and to thereby determine that the mobile device has moved out of wireless range of the wireless transmitter; and to form departure recordal data, the departure recordal data being conveyed to the main computing apparatus.

9. Monitoring apparatus according to any one of the preceding claims wherein when the mobile device is within wireless range of the wireless transmitter the mobile device is operative to determine distance between the mobile device and the wireless transmitter, distance being determined in dependence on strength of wireless signal received by the mobile device from the wireless transmitter.

10. Monitoring apparatus according to any one of the preceding claims wherein the mobile device is configured to convey recorded attendances to the main computing apparatus by way of a wide area wireless communication channel, the mobile device being configured to convey each recorded attendance to the main computing apparatus along with an identifier which is unique to at least one of the mobile device and the worker.

11. Monitoring apparatus according to any one of the preceding claims wherein the main computing apparatus comprises a database containing an attendance schedule for at least one worker, the main computing apparatus being configured to compare recorded attendances received from a mobile device with the attendance schedule and to make a determination in respect of compliance with the attendance schedule in dependence on the comparison.

12. Monitoring apparatus according to any one of the preceding claims wherein the wireless transmitter comprises relative position detecting apparatus, the wireless transmitter being operative in dependence on an output from the relative position detecting apparatus: to record a first location of the wireless transmitter as a reference location; to determine a second location of the wireless transmitter following movement of the wireless transmitter from the first location; and to determine moved location data in dependence on the first and second locations, the data packet transmitted by the wireless transmitter comprising the moved location data.

13. Monitoring apparatus according to claim 12 wherein the mobile device is configured to convey the recorded attendance to the main computing apparatus when the moved location data corresponds to movement of the wireless transmitter by more than a predetermined distance value.

14. Monitoring apparatus according to any one of the preceding claims being care service monitoring apparatus.

15. A method for monitoring remote workers, the method comprising:
providing each of plural short range wireless transmitters at a respective different location remote from main computing apparatus, each short range wireless transmitter being configured to transmit time spaced data packets; and
carrying of each of plural mobile devices by a worker to within wireless communication range of one of the plural wireless transmitters, each of the mobile devices being configured for short range wireless reception of data packets from each of the wireless transceivers, each of the mobile devices being further configured: to record attendance of a worker at the location without worker operation of the mobile device and in dependence on the mobile device being carried by the worker to within wireless communication range of the wireless transmitter at the location whereby the mobile device receives at least one data packet from the wireless transmitter; and to convey each recorded attendance to the main computing apparatus.
